Europäisches Patentamt

European Patent Office

Office européen des brevets

(19)

(11) Publication number: **0 035 882**
**B1**

(12) **EUROPEAN PATENT SPECIFICATION**

(45) Date of publication of patent specification: **04.07.84**

(21) Application number: **81300937.0**

(22) Date of filing: **06.03.81**

(51) Int. Cl.³: **A 23 K 1/18, A 23 K 1/175,**
**A 23 L 1/32, A 61 K 35/54**

(54) Method for producing iodine-enriched eggs.

(30) Priority: **07.03.80 JP 28004/80**
**28.03.80 JP 39054/80**

(43) Date of publication of application:
**16.09.81 Bulletin 81/37**

(45) Publication of the grant of the patent:
**04.07.84 Bulletin 84/27**

(84) Designated Contracting States:
**AT BE CH DE FR GB IT LI LU NL SE**

(56) References cited:
**CH - A - 621 243**
**DE - B - 1 692 489**

**CHEMICAL ABSTRACTS, vol. 52, no. 1, January 10, 1958 abstract 522a Columbus, Ohio, USA I. SUNDE "Iodine content of eggs form hens fed with seaweed meal"**

The file contains technical information submitted after the application was filed and not included in this specification

(73) Proprietor: **NIHON NOSAN KOGYO K.K.**
**No. 2, Shin-urashima-cho, 2-Chome Kanagawa-ku**
**Yokohama-shi Kanagawa-ken (JP)**

(72) Inventor: **Katamine, Shinichiro**
**5-34, No. 1, Sobudai-danchi 1-chome**
**Sagamihara-shi Kanagawa-ken (JP)**
Inventor: **Horiuchi, Mitsuyuki**
**No. 1-8, Takeyama 3-chome Midori-ku**
**Yokohama-shi Kanagawa-ken (JP)**
Inventor: **Sekimoto, Kunitoshi**
**No. 15-2, Sobudai 2-chome**
**Sagamihara-shi Kanagawa-ken (JP)**
Inventor: **Mamiya, Yoneji**
**No. 2-20, Sengen-chi**
**Hiratsuka-shi Kanagawa-ken (JP)**

(74) Representative: **Shipton, Gordon Owen et al,**
**W.P. THOMPSON & CO. Coopers Building Church Street**
**Liverpool L1 3AB (GB)**

Courier Press, Leamington Spa, England.

(56) References cited:

**CHEMICAL ABSTRACTS, vol. 52, no. 6, March 25, 1958, abstract 4763b Columbus, Ohio, USA H. BERGNER "Influence of feeding iodine on egg production, iodine metabolism, and the iodine content of hen eggs"**

**CHEMICAL ABSTRACTS, vol. 53 no. 19, October 10, 1959 abstract 18211f Columbus, Ohio, USA J. VELTHUIZEN: "The excretion of iodine in the hen egg"**

**CHEMICAL ABSTRACTS, vol. 57, no. 7, October 1, 1962, abstract 8987c Columbus, OHIO, USA G.P. GUREVICH: "Iodine concentration in eggs in supplementary feeding of hens with seaweeds and fish meal"**

## Description

This invention relates to an iodine-enriched food product which has the effect of improving lipid metabolism by heightening lipoprotein lipase activity, by reducing glycogen consumption, and by improving the constitution ratio of high-density lipoprotein in the blood.

Americans, as well as Japanese, have been consuming fewer and fewer eggs in recent years. This trend seems to reflect a fear that eating too many eggs may cause heart trouble, a fear based on the theory that maintaining a diet high in cholesterol may increase the amount of serum or hematic cholesterol, which in turn may increase the incidence of coronary artery disease.

This view seems to prevail despite reports that an ordinary diet with one to three eggs per day for several months makes no significant difference in the average level of blood cholesterol. (FEEDSTUFF, August 7, 14, 22 and 24, 1978). Limiting egg ingestion has never been proved to be an effective means of preventing heart disease but the nutritive value of eggs is well known.

However, many recent studies argue that the problem of blood cholesterol relates not as much to the overall quantity of cholesterol as to the kinds of cholesterol and their respective amounts in the blood. The generally-accepted view maintains that a high level of low-density lipoprotein (LDL) will tend to encourage the development and aggravation of heart disease, while a high level of high-density lipoprotein (HDL) will tend to discourage it.

Increasing the level of HDL while lowering the level of LDL is desirable. Ingestion of ordinary egg yolk has been found to slightly increase the level of HDL but it also greatly increase the level of LDL. (Nihon University Medical Department Bulletin #38, 1979).

A number of diets (balancing polyunsaturated and saturated fats, for example) have been devised to heighten the level of HDL in order to prevent hypercholesterolemia. However, many of these have not proved satisfactory because they require major and difficult changes in the patient's eating habits. The food product of this invention can be ingested easily as an ordinary food in a daily diet and can improve the constitution ratio of HDL in the blood. By "constitution ratio" we mean the ratio of HDL cholesterol to total cholesterol.

We have conducted numerous studies on how micronutrients, added to chicken feed, might influence the chemical composition of the egg. We have also studied the effects which different egg compositions have on human diseases e.g. cancer and on lipid metabolism and hematic cholesterol. We have found that ingestion of eggs containing a high amount of iodine has the effect of heightening lipoprotein lipase activity, of reducing glycogen consumption, and of simultaneously improving the constitution ratio of high-density lipoprotein (HDL) in the blood.

Our research has not disclosed any report that any food product containing a high amount of iodine or iodine itself demonstrates the above effects. Our invention, therefore, may be called a "pioneer invention".

The iodine-enriched eggs may be obtained for example in the following manner:— an iodine compound, and a seaweed containing a high amount of iodine, or processed products thereof, is mixed in excess of the "normal" quantity into the feed of egg-laying birds e.g. hens or quails. These birds having been fed or dosed with their feed then lay eggs which are high in iodine content. Iodine compounds which can be used are, for example, calcium iodate; potasium iodate; sodium iodate; potassium iodide; sodium iodide; cuprous iodide; thymol iodide; calcium iodobehemate; iodide salicylic acid; calcium periodate. Examples of seaweed are sea tangle and kelp.

Our studies show that having regard to the health of the bird, chemical stability, and rate of iodine transferability to the egg, calcium iodate is the most desirable iodine compound. It is even more desirable when the seaweed is added to it.

The iodine dosage varies according to the species of egg-laying bird. For hens, the dosage is from 5 to 250, preferably from 5 to 15, mg per day per head. For a hen which ingests approximately 100 g of feed per day, the iodine compound should be added to raise the iodine content of the feed for example 50 to 2,500 ppm, preferably 50 to 150 ppm. After approximately one week, the hen begins to produce eggs containing a high level of iodine. Ingesting a feed containing 50 ppm of iodine yields eggs containing approximately 300 $\mu$g iodine each and a feed containing 100 ppm of iodine yields eggs containing approximately 600—800 $\mu$g each.

By contrast, ordinary egg production requires a feed with 0.30 to 0.35 mg of iodine per kg of feed. According to the American Egg Board, a commercially available feed which contains from 0.3 to 2.0 mg of iodine per kg will produce ordinary eggs containing approximately 6 $\mu$g of iodine each, or at most 30 $\mu$g each. In comparison, the eggs obtained by the methods of this invention contains a particularly high level of iodine.

The iodine-enriched eggs may be used in their natural, non-processed form. They may also be used after being processed, e.g. by heating, drying, concentrating, powdering, or granulating. They may be used for example in the form of granules tablets, or powder mixed with a bonding agent, or in the form of syrup or emulsion.

The invention is illustrated by the following Examples.

## Example 1

Calcium iodate was added to a commercially available feed until its iodine reached 2,000 ppm, and powdered sea tangle was added at the rate of 1%. The feed was fed to 100 hens which had begun to lay eggs seven months earlier. A total of 170 kg of eggs produced by this method were spray-dried, whereby 35 kg of dried egg were obtained. This powdered egg had an iodine content of 411 mg per kg.

## Example 2

Diiodo salicylic acid was added to a commercially available feed to obtain an iodine content of 100 ppm, and powdered sea tangle was added at the rate of 1%. The feed resulted in eggs with an average iodine content of 700 μg each.

## Example 3

Sodium iodate was added to a commercially available hen food so that the iodine content became 100 ppm, and powdered seaweed was added at the rate of 0.5% resulting in eggs containing on average 650 μg of iodine per egg on feeding to egg-laying hens as in Example 2.

To ascertain the safety of the food product of the invention the following test was conducted.

## Acute virulence test

Animals used: 10 female rats, Wistar descent, weighing 100—110 g each.

In this test, rats were fed iodine enriched powdered eggs in doses which were increased in geometric ratio to the maximum of 50 mg of iodine per kg of the rat's weight. After seven days, none of the rats had died or lost weight. The product therefore had no acute virulence.

The following animal experiments were then conducted to observe the effect of the food product of the invention in improving the constitution ratio of high-density lipoprotein (HDL) in the blood.

## Experiment 1

Animals used: 30 male rats, SD descent. Three-week-old rats were placed in cages with a voluntary exercise apparatus. For 10 days they were fed a commercially available solid basic feed. They were fed twice a day between 8 and 9 a.m. and between 9 and 10 p.m. with a powdered basic feed made by Nihon Nosan Kogyo, K.K. until they were 7 weeks old. They were divided into two groups of 15 each, with the same average weight and performance of voluntary exercise. The first group, designated as the test group, was fed a test feed which had been prepared by adding 2% powdered iodine enriched egg of Example 1 to the powdered basic feed. The second group, designated as the control group, was fed a control feed which has been prepared by adding 2% powdered ordinary egg to the powdered basic feed. Tables 1 and 2 show the composition of egg powders and feed, respectively.

TABLE 1

|  | Example 1 powdered egg | Ordinary powdered egg |
|---|---|---|
| % Moisture | 4.2 | 3.6 |
| % Crude protein | 48.5 | 49.6 |
| % Crude fat | 40.9 | 40.3 |
| % Crude ash | 3.9 | 4.5 |
| % Calcium | 0.25 | 0.25 |
| % Phosphorus | 0.88 | 0.89 |
| % Potassium | 0.52 | 0.52 |
| % Magnesium | 0.19 | 0.19 |
| mg Iodine/kg | 411.0 | 3.0 |

TABLE 2

|  | Test group | Control group |
|---|---|---|
| % Moisture | 11.8 | 11.1 |
| % Crude protein | 19.3 | 19.4 |
| % Crude fat | 4.8 | 4.9 |
| % Crude fiber | 6.3 | 6.7 |
| % Crude ash | 6.0 | 6.1 |
| μg Protein/100 g | 816.3 | 36.9 |

The temperature in each feeding cage was maintained at 25°±2°C and humidity, between 50% and 80%. The amount of each feed ingested was measured for three days per week, and each rat was weighed twice per week. The rats were fed in this manner for 10 weeks and then were decapitated some at 5 p.m., some at 9 p.m., some at 12 a.m., or before, during and after feed ingestion to measure the following: lipoprotein lipase activity in the adipose tissue; lipoprotein lipase activity in the skeletal

4

muscles; glycogen level in the liver; glycogen level in the skeletal muscles; total amount of whole blood cholesterol; amount of hematic high-density lipoprotein (HDL); and constitution ratio of hematic HDL.

Although there was little difference between the test group and the control group with respect to the amount of feed ingestion and weight increase (Table 3), the amount of voluntary exercise of the test group was consistently higher than that of the control group.

TABLE 3

| Average value of 15 heads | Test group | Control group |
|---|---|---|
| Feed ingestion amount g/head/10 weeks | 1,694.1 | 1,725.8 |
| Weight increase g/head/10 weeks | 188.9 | 202.5 |

Tables 4 and 5 show measurement of lipoprotein lipase (LPL) activity and the level of glycogen, respectively.

TABLE 4

(Enzymatic reaction was determined by Gasquet's method; the amount of isolated fatty acid was then determined by Dole's method as improvided by Trout et al.)

| Average value of 4—5 heads LPL activity in adipose tissue | Test group | Control group |
|---|---|---|
| 5 p.m. | 25.9 | 25.1 |
| 9 p.m. | 20.2 | 14.9 |
| 12 a.m. | 31.5 | 24.1 |

| LPL activity in skeletal muscles | Test group | Control group |
|---|---|---|
| 5 p.m. | 23.6 | 16.4 |
| 9 p.m. | 29.5 | 26.3 |
| 12 a.m. | 27.4 | 22.2 |

TABLE 5

(The amount of glycogen was determined by the method of Lo et al).

| Average value glycogen in liver | Test group | Control group |
|---|---|---|
| 5 p.m. | 46.9 | 40.5 |
| 9 p.m. | 43.0 | 32.1 |
| 12 a.m. | 44.2 | 44.0 |

| Glycogen in skeletal muscles | Test group | Control group |
|---|---|---|
| 5 p.m. | 4.10 | 3.81 |
| 9 p.m. | 4.22 | 3.20 |
| 12 a.m. | 5.40 | 4.33 |

The measurements of the constitution ratio of hematic high-density lipoprotein (HDL) is shown in Table 6.

TABLE 6

(The total amount of whole cholesterol was determined by Zurkowski's direct coloration-ultra-microanalysis method; the percentage of HDL was determined with subsequent use of the heparin $Mn^{2+}$ processing method of Warnick et al.).

| Average value of 14 heads | Test group | Control group |
|---|---|---|
| (A) Whole cholesterol mg/dl | 73.6 | 81.7 |
| (B) HDL cholesterol mg/dl | 58.5 | 62.2 |
| Ratio of HDL or B/A | 79.9% | 76.1% |

Experiment 2

Animals used: 26 male rats, SD descent

3-Week-old rats were fed in the same manner as in Experiment 1, but the cages had no voluntary exercise apparatus. Instead, the rats were compelled to exercise once a week with a compulsory swimming exercise apparatus so as to experience fatigue. The apparatus was set in 30°C water with approximately 40 m/min of current speed.

The first group was composed of 14 rats, the second, 12. The total amount of whole cholesterol and the amount of hematic HDL of each group were determined as in Experiment 1. These measurements are shown in Table 7. It is apparent that the constitution ratio of hematic HDL was improved by the food product of the invention.

TABLE 7

| Average value | Test group | Control group |
| --- | --- | --- |
| (A) Whole cholesterol mg/dl | 85.8 | 84.0 |
| (B) HDL cholesterol mg/dl | 72.1 | 66.6 |
| Ratio of HDL or B/A | 83.4% | 79.9% |

After the safety of the food product and is effect in improving the constitution ratio of hematic HDL in animals had been ascertained, clinical tests were conducted.

Clinical test

A daily dose of 3 g of powdered iodine enriched egg of Example 2 was given over a period of 2 to 3 months to 10 outpatients with hypercholesterolemia. Both before and after dosage ingestion, the total amount of whole blood cholesterol, the level of hematic HDL, and the level of hematic LDL were measured (Table 8). The food product increased the amount of HDL while lowering that of LDL.

TABLE 8

| | Before dosage (mg/dl) | After dosage (mg/dl) |
| --- | --- | --- |
| Whole cholesterol | | |
| Patient 1 (male) | 247 | 241 |
| 2 (male) | 260 | 222 |
| 3 (male) | 209 | 224 |
| 4 (female) | 369 | 369 |
| 5 (male) | 241 | 236 |
| 6 (male) | 213 | 219 |
| 7 (male) | 208 | 222 |
| 8 (female) | 308 | 307 |
| 9 (male) | 320 | 318 |
| 10 (male) | 215 | 237 |
| HDL Cholesterol | | |
| Patient 1 (male) | 43 | 61 |
| 2 (male) | 36 | 38 |
| 3 (male) | 33 | 38 |
| 4 (female) | 33 | 82 |
| 5 (male) | 31 | 40 |
| 6 (male) | 52 | 66 |
| 7 (male) | 54 | 56 |
| 8 (female) | 68 | 87 |
| 9 (male) | 35 | 35 |
| 10 (male) | 43 | 59 |
| Ratio of HDL | | |
| Patient 1 (male) | 17.4% | 25.3% |
| 2 (male) | 13.8% | 17.1% |
| 3 (male) | 15.8% | 17.0% |
| 4 (female) | 8.9% | 22.2% |
| 5 (male) | 12.9% | 16.9% |
| 6 (male) | 24.4% | 30.1% |
| 7 (male) | 26.0% | 25.2% |
| 8 (female) | 22.1% | 28.3% |
| 9 (male) | 10.9% | 11.0% |
| 10 (male) | 20.0% | 24.9% |

LDL

| Patient | | | |
|---------|---|-----|-----|
| Patient 1 (male) | | 581 | 562 |
| 2 (male) | | 631 | 530 |
| 3 (male) | | 432 | 509 |
| 4 (female) | | 972 | 903 |
| 5 (male) | | 548 | 557 |
| 6 (male) | | 395 | 456 |
| 7 (male) | | 558 | 498 |
| 8 (female) | | 705 | 704 |
| 9 (male) | | 773 | 773 |
| 10 (male) | | 450 | 512 |

Both animal experiments and clinical tests indicate that the food product of the invention heightens lipoprotein lipase activity and reduces glycogen consumption, and improves the constitution ratio of hematic HDL.

Although the precise cause of this has not yet been determined, it is theorized that the iodine transferred to eggs through the hen's living body combines chemically with the composition of the egg, and that this combination directly or indirectly acts on lipoprotein lipase activity to produce said effects.

Continued ingestion of the food product is desirable because it does not produce immediate effects. The product does not cause any ill side-effects, and it is superior to ordinary eggs in its nutritive value.

When one or two eggs with an iodine content of 300 to 1,000 $\mu$g per egg are ingested as a part of one's daily diet, the intended improvement begins to show in approximately one month. This is accomplished without abnormally upsetting the constitution ratio of the hematic lipoproteins.

It is desirable to ingest the product of the invention continuously because, as stated above, it is slow acting.

## Claim

A method for producing an iodine-enriched egg containing at least 300 $\mu$g of iodine characterised in that an egg-laying bird is fed with an iodine compound and seaweed containing iodine in such an amount that the bird receives 5 to 250 mg of iodine per day.

## Revendication

Méthode pour produire un oeuf enrichi en iode contenant au moins 300 microgrammes d'iode, caractérisée en ce que l'oiseau pondeur est nourri avec un composé de l'iode et une algue contenant de l'iode en une quantité telle que l'oiseau reçoive 5 à 250 mg d'iode par jour.

## Patentanspruch

Verfahren zur Herstellung eines mit Jod angereicherten Eies, das mindestens 300 $\mu$g Jod enthält, dadurch gekennzeichnet, daß ein eierlegender Vogel mit einer Jodverbindung und Jod enthaltendem Seetang in solcher Menge gefüttert wird, daß der Vogel pro Tag 5 bis 250 mg Jod erhält.